# EUROPEAN PATENT APPLICATION

(11) **EP 4 290 218 A1**
(43) Date of publication of application: **13.12.2023**
(21) Application number: 22177515.8
(22) Date of filing: 07.06.2022
(51) Int. Cl.: G01N 21/33, G01N 21/3504, G01M 15/10, G01N 33/00, G01N 21/31, G01N 21/39, G01N 21/17

(54) **EQUIPMENT FOR MEASURING POLLUTANT EMISSIONS ON MULTI-LANE ROADS EMITTED BY MOTOR VEHICLES BY MEANS OF LASER SPECTROSCOPY USING LIGHT INTENSITY ABSORPTION DEVICES**

(71) Applicant: de la Fuente Egido, Josefina, 28045 Madrid (ES)
(72) Inventor: de la Fuente Egido, Josefina, 28045 Madrid (ES)
(74) Representative: Lahidalga de Careaga, Jose Luis

(57) **Abstract**

Equipment for measuring pollutant emissions on multi-lane tracks emitted by motor vehicles by laser spectroscopy using light intensity absorption devices that work on the track from top to bottom, measuring the pollutant emissions emitted by motor vehicles, by reflecting the light from top to bottom and with information collected by sensors with FPGA electronics located on the ground of the track. The methodology of the proposed equipment consists of performing top-down measurements from a source cabinet located on top of a gantry on the track using laser spectroscopy and integrating: quantum cascade lasers (6), to measure CO, NH3, NO and NO2 emissions; ICL laser (7), to measure HC and a UV light source (8) at 230 nm to measure opacity.

## Description

### OBJECT OF THE INVENTION

The object of the invention, as is clear from the title, consists of a device for measuring pollutant emissions on multi-lane roads emitted by motor vehicles by laser spectroscopy using light intensity absorption devices that work on the road from top to bottom, measuring the pollutant emissions emitted by motor vehicles, by means of the reflection of light from top to bottom and with information collected by sensors located on the ground of the road.

The methodology of the proposed equipment is to perform top-down measurements on the road using laser spectroscopy.

Quantum cascade lasers are usually used either by absorption of laser light or by laser scattering of pollutants emitted by motor vehicles on roads or similar, where the active elements, such as lasers, detectors, computer power supply and so on, are located and accessible at road level and the detector modules are embedded in the asphalt of the road.

### BACKGROUND OF THE INVENTION

With the increasing use of cars, trucks, aircraft and other combustion engine vehicles, concern about the pollutant gases emitted by these sources is justifiably growing.

Carbon monoxide, the toxic by-product of incomplete combustion, is the largest contributor to air pollution and poses a very real threat to public health.

Carbon dioxide, while not toxic, is recognized as an air pollutant that directly causes the "greenhouse effect". Modern fuels generate excessive amounts of carbon dioxide which, according to scientists, are polluting the global atmosphere.

Today's engines also generate a quantity of nitrogen oxides, ammonia, micro-particulates and other types of pollutant compounds harmful to health, which are generally responsible for eye irritation, nasal congestion and respiratory difficulties.

Numerous devices and methods for the control of exhaust pollutants are known in the state of the art.

Electrostatic precipitation is widely used in such applications and involves the application of high voltages to electrodes located in the exhaust gas stream. The process induces ionisation of the gaseous particles, which, in turn, causes the particles suspended in the gas to acquire a charge through contact with the ionised particles in the gas. The charged particles are then collected on oppositely charged electrodes, which must eventually be purified.

A significant drawback of electrostatic precipitation is that only minute particulate matter can be precipitated out of the exhaust stream. The process is ineffective in removing gaseous pollutants such as carbon monoxide and carbon dioxide.

Remote sensing systems have been under development for many years. For example, US patent US5210702 discloses a system for remotely detecting carbon monoxide and carbon dioxide levels in vehicle emissions. The system uses an infrared (IR) source to project a collimated beam of IR radiation through the exhaust plume of a passing vehicle. An optical device is used to separate various wavelengths of the beam and direct them to particular photo-detectors. Each photo-detector generates an electrical signal based on the presence or absence of light of a specified wavelength. The electrical data is entered into a computer which is used to calculate and compare the rates of carbon monoxide and carbon dioxide exhaust components. From these rates, high emitting vehicles are identified. These rates can also be entered into a series of equations based on stoichiometric ratios between exhaust components that are used to compute the concentrations that would be observed by a tailpipe probe (with correction for water and any excess air).

Other methods of determining emission concentrations using remote optical gas analyzers have also been attempted. One method, disclosed in US patent US4924095, uses multiple beam paths to sample a cross-sectional "slice" of the exhaust plume. The volume of the slice is determined and used to calculate an absolute concentration of one or more exhaust components. Such a system proved to be inaccurate and unfeasible in practice due to the uneven dispersion of the exhaust plume and difficulties emissions when calculating the exhaust plume volume.

Remote vehicle emissions testing systems have undergone many improvements since they were originally released. Examples include: monitoring with associated video of the vehicle whose emissions are to be analyzed and number plate readers to actually "read" the number plate; the combination of UV and IR radiation sources for detectors with CO, CO2, NOx, water, and hydrocarbon detector channels; and various optical arrangements that perform beam decomposition, beam paths, filtering, and multi-plexing over time.

Although cars and trucks are the largest source of pollutant vehicle emissions, vehicles with smaller engines, such as motorbikes, mopeds and other small motorised vehicles can also contribute to the accumulation of pollutants in urban areas. Because small motor vehicle engines typically generate considerably smaller and less dense exhaust plumes, current remote sensing systems for cars and trucks may have difficulty distinguishing exhaust readings for small motor vehicles from background noise. For example, a 50 cc moped produces an exhaust plume ten to twenty times smaller than that of a small car. In addition, the spatial location of small engine exhaust plumes can be critical for successful remote detection due to their small size and rapid dispersion. Due to the variability in the height of motorbike exhaust outlets, a motorbike exhaust plume can occur anywhere from 6 inches to 3 feet above the ground. Current remote sensing systems can have difficulty targeting the exhaust plume of vehicles with small engines and exhaust outlets of varying height.

US 5877862 discloses a system for analyzing exhaust gases using tunable infrared spectroscopy. A laser beam is directed through an exhaust plume, reflected by a reflector and then received by a detector after a second pass through the exhaust plume.

US 4924095 discloses an optical system for measuring exhaust pollution from cars and trucks, discussing the possibility of multiple beam reflections with respect to figure 6 of that document.

US 5591975 discloses an exhaust gas analysis system in which photo-detectors are sensitive to spectral absorption peaks of CO, CO2, NO, H2O and hydrocarbons. The composition of the plume is calculated as percentages of the constituents based on the detected transmittances of the respective wavelengths.

These and other drawbacks of current remote emission detection systems are solved by one or more of several preferred embodiments of the invention.

The present invention is a novel system which due to its configuration can determine the pollutant components emitted by motor vehicles with such flexibility that it can employ both traditional light absorption techniques and laser scattering spectroscopy techniques, which is based on molecular scattering, detecting changes in the refractive index occurring in the vicinity of a molecular transition, which avoids measurement errors in the procedures known in the state of the art and which by means of quantum cascade lasers can be used to measure CO, NH3, NO and NO2 emissions; by means of ICL lasers (7) measures the level of HC and by means of UV light source (8) at 230 nm it measures the opacity and where the light detector modules, embedded in the asphalt of the road, are made up of FPGA type electronic paths.

Indeed the recommended invention differs in form and manner.

### BRIEF DESCRIPTION OF THE INVENTION

The object of the invention consists of an equipment for measuring pollutant emissions on multi-lane roads emitted by motor vehicles by laser spectroscopy by means of light intensity absorption devices of those working on the road from top to bottom, measuring the pollutant emissions emitted by motor vehicles, by means of the reflection of the light from top to bottom and with information collected by sensors located on the road surface.

The methodology of the proposed equipment is to perform top-down measurements on the road using laser spectroscopy.

Quantum cascade lasers are usually used either by absorption of laser light or by laser scattering of pollutants emitted by motor vehicles on roads or similar, where the active elements, such as lasers, detectors, computer power supply and so on, are located and accessible at the road level and the detector modules are embedded in the road surface.

Detectors, computer power supply and so on, are located and accessible at track level and the detector modules are embedded in the track asphalt.

In this case, quantum cascade lasers are used to measure CO, NH3, NO and NO2 emissions; by means of ICL lasers the level of HC is measured and by means of UV light source at 230 nm the opacity is measured.

The intensity of the lasers is distributed as follows in an example of three lanes of the same track from the top cabinet located on a gantry, which incorporates a fixed lens that reflects the light received in the form of a beam of light to a detector.

These light beams are incident on the detectors which analyses them after passing through the plumes of pollutant emissions from the motor vehicles on the road. The light detector modules, embedded in the asphalt of the road, are made up of FPGA type electronic paths.

The data are calculated in pm concentrations and pollutant-to-CO2 ratios using the CO2 channel as a reference.

The data is either sent to the cloud or sent to a local system.

### DESCRIPTION OF THE DRAWINGS

In order to complement the description being made and with the object of aiding a better understanding of the characteristics of the invention, in accordance with a preferred example of the practical realization thereof, there is attached as an integral part of said description, a set of drawings in which, by way of illustration and not limitation, the following has been represented:

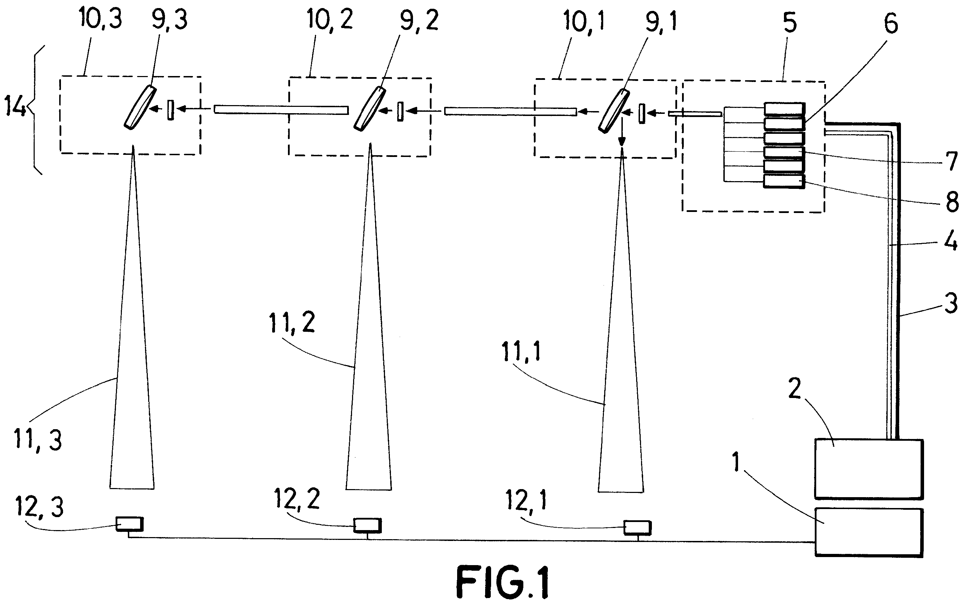

Figure 1. Shows a schematic view of the measuring equipment by means of light partitioning.

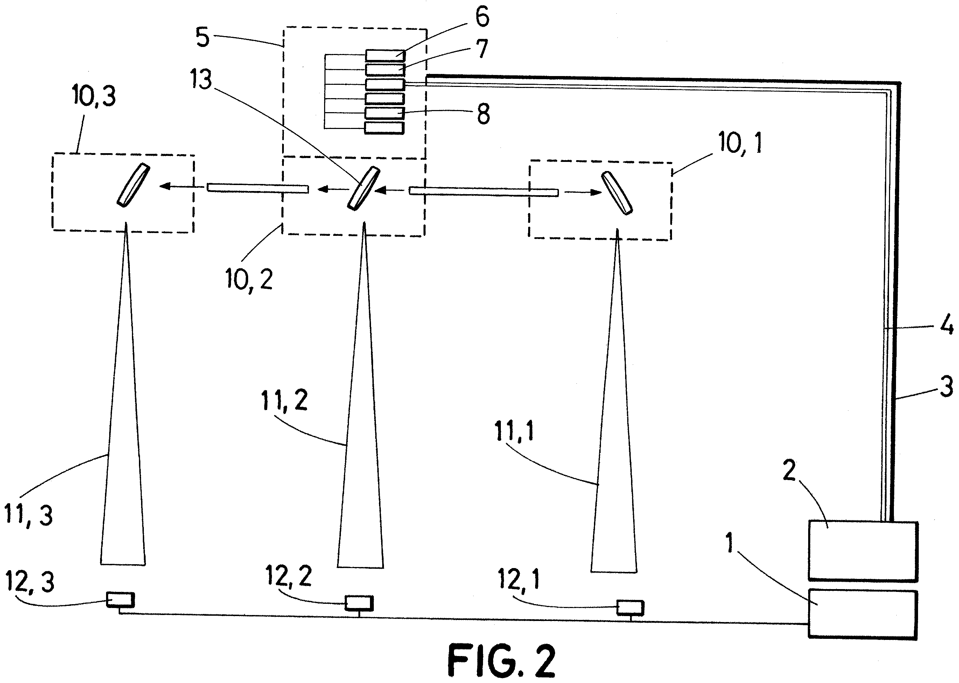

Figure 2. Shows a schematic view of the measuring equipment by means of a Galvo mirror that turns the light to all the detectors.

And in these figures the same elements are identified with identical numbering:
(1). - power suply module,
(2). - computing and communication module,
(3), - power transmission cable,
(4).- communications transmission cable,
(5). - source cabinet,
(6).- quantum cascade lasers,
(7). - ICL laser,
(8).- light source,
(9.1).- fixed lens line 1,
(9.2).- fixed lens line 2,
(9.3).- fixed lens line 3,
(10.1).- top cabinet line 1,
(10.2).- top cabinet line 2,
(10.3).- top cabinet line 3,
(11.1).- light beam line 1,
(11.2).- light beam line 2,
(11.3).- light beam line 3,
(12.1).- detector module line 1,
(12.2).- detector module line 2,
(12.3).- detector module line 3,
(13).- galbo mirror,
(14).- support gantry,

### PREFERRED EMBODIMENT OF THE INVENTION

The equipment for measuring pollutant emissions on multi-lane roads emitted by motor vehicles by laser spectroscopy by means of light intensity absorption devices of those working on the track from top to bottom, measuring the pollutant emissions emitted by reflection of the light from top to bottom and with information collected by sensors located on the ground of the track.

The present invention brings the novelty of using both quantum cascade laser techniques, ICL lasers to measure HC and a UV light source to measure opacity to measure pollutants.

The measurement equipment allows the pollutants emitted by motor vehicles to be determined by a combination of elements placed both at road level and above the road.

More precisely, the equipment comprises a power supply module (1) which supplies energy to the assembly and which is located at track level. In certain cases it could be located above the track on the support gantry (14).

Together with the power supply module (1) there is the computing and communication module (2) which controls the management of the assembly and the communications of the whole apparatus.

These two elements communicate directly with the source cabinet (5) by means of two conductor cables, a transmission power cable (3) and a transmission communication cable (4).

The source cabinet (5), located on top of the gantry (14) houses at least the following elements:
.- Quantum cascade lasers (6), for measuring CO, CO2, NH3, NO and NO2 emissions.
.- ICL laser (7), to measure HC.
.- UV light source (8) at 230 nm to measure opacity.

The intensity of the lasers is distributed as follows in an example of three lanes of the same track from the top cabinets located on the gantry (14). And aligned with the source cabinet (5). top cabinet line 1 (10.1) incorporating a fixed lens line 1 (9.1) which reflects 33% and transmits 66%, of the light received in the form of a light beam (11.1) to the detector line 1 (12.1) located on the track. This line is the closest to the emission output from the source cabinet (5). top cabinet line 2 (10.2) incorporating a fixed lens line 2 (9.2) which reflects 50 per cent of the received light as a beam of light (11.2) to the detector line 2 (12.2) located on the track. top cabinet line 3 (10.3) incorporating a fixed lens line 3 (9.3) which reflects 100 % of the received light as a beam of light (11.3) to the detector line 3 (12.1) located on the track.

These light beams are incident on the detectors which analyse them after passing through the plumes of pollutant emissions from the motor vehicles on the track.

The light detector modules, embedded in the track asphalt, one per line, (12.1), (12.2) and (12.3) are composed of FPGA type electronic paths and have the functions of:
.- synchronising and processing the capture data,
.- generation of gas style,
.- reception of the analogue signal from the IV and IR detectors,
.- monitoring the temperature of the lasers and the box to give the software a protection.

The data is calculated in concentrations of in pm and in ratios of pollutants in percent of CO2 taking the CO2 channel as a reference.

The data is either sent to the cloud or sent to a local system.

In another preferred embodiment the arrangement changes as follows:
The source cabinet (5), located above the gantry (14) is placed on line 2, above a Galvo mirror which turns the light to the cabinet line with a reception M 100% which forwards it to the light detectors embedded in the asphalt.

The nature of the invention having been sufficiently described, as well as the manner of its implementation, it should be noted that the arrangements indicated above and shown in the attached drawings are subject to modifications of detail insofar as they do not alter their fundamental principles, as set out in the preceding paragraphs and summarised in the following claims.

## Claims

1. Equipment for measuring pollutant emissions on multi-lane roads emitted by motor vehicles by laser spectroscopy using light intensity absorption devices that work on the road from top to bottom, measuring the pollutant emissions by means of the reflection of light from top to bottom and with information collected by sensors located on the ground of the road and **characterized in that** the equipment comprises the following elements:
.- a power supply module (1) which supplies power to the assembly and which is housed at track level a computing and communication module (2) which controls the management of the assembly and the communications of the whole apparatus.
.- a source cabinet (5) located above the gantry (14), connected to the above elements by means of two conductor cables, a transmission cable power (3) and transmission cable communication (4) and housing at least the following elements
.- quantum cascade lasers (6), for measuring CO, CO2, NH3, NO and NO2 emissions.
.- ICL laser (7), for measuring HC.
.- UV light source (8) at 230 nm to measure opacity.
- top cabinets located on the gantry (14) aligned with the source cabinet (5) and which on a three-rail track has:
.- a top cabinet line 1 (10.1) incorporating a fixed lens line 1 (9.1) that reflects 33% and transmits 66% of the received light in the form of a beam (11.1) to the detector line 1 (12.1);
the detector line 1 (12.1) located on the track.
.-a top cabinet line 2 (10.2) incorporating a fixed lens line 2 (9.2) reflecting 50% of the light received in the form of a light beam (11.1) to the detector line 1 (12.1) located on the track. reflects 50 % of the received light as a beam of light (11.2) to the detector line 2 (12.2).
line 2 detector (12.2) located on the track.
.- a top cabinet line 3 (10.3) incorporating a fixed lens line 3 (9.3) that reflects 100 % of the received light in the form of a beam of light (11.3) to the line 3 detector (12.1) located on the track.
.- light detecting modules, embedded in the track asphalt, thus one per line, (12.1), (12.2) and (12.3) consisting of FPGA type electronic traces.

2. Equipment for measuring pollutant emissions on multi-lane roads emitted by motor vehicles by laser spectroscopy using light intensity absorption devices that work on the road from top to bottom, measuring the pollutant emissions by means of the reflection of light from top to bottom and with information collected by sensors located on the ground of the road in accordance with claim 1ª and **characterized in that** the light beams (11. 1), (11.2) and (11.3) fall on the detectors (12.1), (12.2) and (12.3) which analyses them after passing through the plumes of pollutant emissions of the motor vehicles circulating on the road.

3. Equipment for measuring pollutant emissions on multi-lane roads emitted by motor vehicles by laser spectroscopy using light intensity absorption devices that work on the road from top to bottom, measuring the pollutant emissions by means of the reflection of light from top to bottom and with information collected by sensors located on the ground of the road in accordance with claim 1 and characterized because the light detector modules, embedded in the asphalt of the road, one per line, (12.1), (12.2), (12.3), (12.4), (12.4), (12.5), (12.6), (12.7), (12.8), (12.8), (12.9) and (12.9). 1), (12.2) and (12.3) consisting of FPGA type electronic paths have the functions of:
.- synchronizing and processing the data of the captures,
.- gas style generation
.- reception of the analogue signal from the IV and IR detectors,
.- monitoring of the temperature of the lasers and the box to give the software a protection.

4. Equipment for measuring pollutant emissions on multi-lane roads emitted by motor vehicles by laser spectroscopy using light intensity absorption devices that work on the road from top to bottom, measuring the pollutant emissions by means of the reflection of light from top to bottom and with information collected by sensors located on the ground of the road in accordance with claim 1ª and characterized because the data is calculated in concentrations of pm and in ratios of pollutants between so many percent of CO2 taking the CO2 channel as a reference.

5. Equipment for measuring pollutant emissions on multi-lane roads emitted by motor vehicles by laser spectroscopy using light intensity absorption devices that work on the road from top to bottom, measuring the pollutant emissions by means of the reflection of light from top to bottom and with information collected by sensors located on the ground of the road in accordance with claim 1, **characterized in that** the data is sent to the cloud or to a local system.

6. Equipment for measuring pollutant emissions on multi-lane roads emitted by motor vehicles by laser spectroscopy using light intensity absorption devices that work on the road from top to bottom, measuring the pollutant emissions by means of the reflection of light from top to bottom and with information collected by sensors located on the ground of the road in accordance with claim 1 and characterized because in an alternative embodiment the source cabinet (5), located above the gantry (14) is placed on line 2, above a Galvo mirror that turns the light to the cabinet line with a reception M 100% that forwards it to the light detectors embedded in the asphalt.
